Europäisches Patentamt

(19) European Patent Office

Office européen des brevets

(11) Publication number: **0 102 141**
**B1**

(12) **EUROPEAN PATENT SPECIFICATION**

(45) Date of publication of patent specification: **20.11.86**

(21) Application number: **83302719.6**

(22) Date of filing: **13.05.83**

(51) Int. Cl.⁴: **A 61 M 5/16,** A 61 M 5/14

(54) Apparatus for administering a liquid to patients.

(30) Priority: **26.05.82 GB 8215439**
**07.02.83 GB 8303270**

(43) Date of publication of application:
**07.03.84 Bulletin 84/10**

(45) Publication of the grant of the patent:
**20.11.86 Bulletin 86/47**

(84) Designated Contracting States:
**CH DE FR GB IT LI SE**

(56) References cited:
**DE-B-2 264 148**
**US-A-2 784 733**
**US-A-2 844 147**
**US-A-4 078 563**
**US-A-4 175 558**

(73) Proprietor: **WEST PHARMARUBBER LIMITED**
**Bucklers Lane**
**St. Austell Cornwall, PL25 3LP (GB)**

(72) Inventor: **Stafford, Peter William**
**'Bosinney' Polgooth**
**St. Austell Cornwall (GB)**

(74) Representative: **Wilson, Joseph Martin et al**
**WITHERS & ROGERS 4 Dyer's Buildings Holborn**
**London EC1N 2JT (GB)**

## Description

This invention is concerned with the provision of improved apparatus for use in the administration of liquid to the body of a patient, including safety means positively to cut off the flow of liquid when the liquid in the apparatus reaches a predetermined level.

There is currently on the market an apparatus for administering liquid directly into the body, known under the common name of a 'Burette Set'. Such administration devices are available from several of the major Pharmaceutical Companies. That device is used to give a large volume of infusion liquid over a period of time and consists of a plastics burette tube of approximately 38.1 mm (1½") diameter and some 152.4 mm (6") in length which is graduated along its length. The liquid in question enters the top of the tube and is administered from the base of the tube through an infusion line into the body. The graduations on the tube monitor the dosage. It is of vital importance that when the liquid level approaches the bottom of the burette tube there is a known cut-off point when the liquid will cease to flow and air will enter the line, as air in any infusion line is a major hazard. There are several designs of valve at the base of the tube for ensuring that the liquid cuts off at the correct level and does not admit air.

"US—A—4 175 558 describes such a burette apparatus, for administering liquid into the body of a patient, which apparatus comprises the features specified in the prior art portion of claim 1. The valve includes a buoyant float ball and a valve seat positioned about the bottom flow aperture to receive the ball, in the absence of sufficient liquid to float it, in air-tight, sealing relation. Since it is not possible to ensure that every ball is perfectly spherical, a leakage problem may arise".

According to the present invention we provide burette apparatus, for administering liquid into the body of a patient, comprising a burette tube into which liquid can be fed at an upper end and from which liquid can flow out at a lower end into the entrance opening of a thin hollow cannula, the tube being provided with a cup shaped projection forming part of the bottom of the tube, and through which liquid can flow out of the tube into the cannula, a floatable valve member within the burette tube and means to restrict the upward floating movement of the valve member so that in its upper position it is still in the lower part of the tube, the arrangement being such that when the level of the liquid in the tube falls to a predetermined level the valve member in its lower position stops the flow of liquid into the cannula by sealing the entrance to the cannula within the cup shaped projection, characterised in that the floatable valve member comprises a substantially cylindrical valve body which fits within the burette tube leaving an annular space between the outer edge of the member and the inner surface of the tube whereby the cross-sectional area of the valve body is substantially greater than that of the hollow cannula, and in that the valve member has a depending valve closing part including a rubber or other resilient sealing member which is substantially flat on the underside and which is operable to close the entrance opening of the hollow cannula with the resilient sealing member resting directly on the top of the cannula so that the flat under-surface of the sealing member extends horizontally across the entrance opening of the cannula.

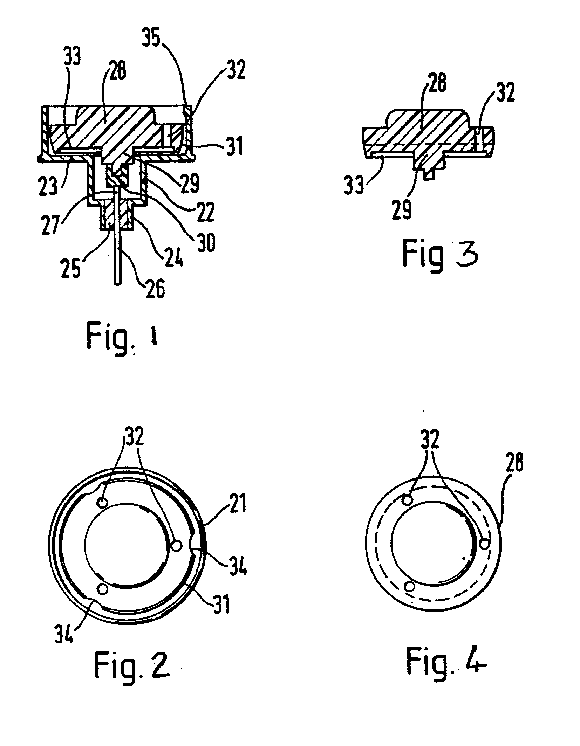

An embodiment of the invention is illustrated by way of example in Figures 1 to 4 in which Figure 1 is a sectional view of a floating valve in position in a burette tube, Figure 2 is a planview of Figure 1.

Figure 3 is a sectional view of the valve head, and Figure 4 is a planview of Figure 3.

In the drawings, a hollow burette tube 21 has a downwardly extending cup-shaped projection 22 at the bottom 23 with a central exit opening 24 closed by means of a moulded or like plug 25 into which a thin hollow cannula 26 is fitted. The cannula 26 is open at the top and bottom so that liquid within the tube 21 can flow into the cannula, the upper end of which projects at 27 above the top of the plug 25.

A floatable valve member 28 is disposed above the cup-shaped projection and the member 28 has a depending valve closing part 29 provided with a rubber or other suitable resilient or the like sealing member 30. The outer diameter of the member 28 is less than the inner diameter of the tube 21 so that there is an annular space 31 between the member 28 and the tube 21. In addition the member 28 has holes 32 leading from the top of the member 28 to an undercut portion 33 at the bottom of member 28. The member 28 also has peripheral recesses 34 to cooperate with spaced apart projections 35 on the inside of the tube 21.

The valve member 28 is assembled within the tube 21 by passing the member 28 down from the top of the tube in such a way that the recesses 34 can pass the projections 35. The member 28 is then angularly displaced so that in operation rising of the member 28 is controlled by the projections 35. When the valve member is in its down position the sealing member 30 is resting on the top of the cannula 26 so that the exit from the tube 21 is closed. If liquid be fed into the tube 21 the valve member will rise until the top of the member 28 contacts the projections 35 so that liquid can flow down the holes 32 and through the space 31 into the cup-shaped projection 22 and from there into the cannula 26 because rising of the member 28 has moved the sealing member 30 away from the top of the cannula.

It will be noted that the actual closing operation is effected over a very small area ie between the sealing member 30 and the cannula 26. The small area sealing has advantages in certain cases because the possibility of leakage is reduced.

If the liquid level in the tube 21 falls below a safe level, the valve member 28 will return to its down position, the member 30 will seat on the top of the cannula 26 and flow of liquid into the cannula will stop.

We prefer that the valve member 28 shall be made of a suitable plastics material and that the sealing member 30 shall be made of rubber so that a rubber to cannula seal is achieved.

It will be understood that the member 28 may have an uninterrupted periphery which can simply be pushed over the projections 35 to locate the member 28.

**Claims**

1. Burette apparatus, for administering liquid into the body of a patient, comprising a burette tube (21) into which liquid can be fed at an upper end and from which liquid can flow out at a lower end into the entrance opening of a thin hollow cannula (26), the tube (21) being provided with a cup shaped projection (22) forming part of the bottom (23) of the tube and through which liquid can flow out of the tube (21) into the cannula (26), a floatable valve member (28) within the burette tube (21) and means (35) to restrict the upward floating movement of the valve member (28) so that in its upper position it is still in the lower part of the tube (21), the arrangement being such that when the level of the liquid in the tube (21) falls to a predetermined level the valve member (28) in its lower position stops the flow of liquid into the cannula (26) by sealing the entrance to the cannula (26) within the cup shaped projection (22), characterised in that the floatable valve member (28) comprises a substantially cylindrical valve body which fits within the burette tube (21) leaving an annular space (31) between the outer edge of the member (28) and the inner surface of the tube (21) whereby the cross-sectional area of the valve body is substantially greater than that of the hollow cannula (26), and in that the valve member (28) has a depending valve closing part (29) including a rubber or other resilient sealing member (30) which is substantially flat on the underside and which is operable to close the entrance opening of the hollow cannula (26) with the resilient sealing member (30) resting directly on the top of the cannula (26) so that the flat undersurface of the sealing member (30) extends horizontally across the entrance opening of the cannula (26).

2. Apparatus according to Claim 1 wherein the cup-shaped projection (22) has a central exit opening (24) closed by means of a moulded or like plug (25) into which the thin hollow cannula (26) is fitted, the cannula (26) being open at the top and bottom so that liquid within the tube (21) can flow into the cannula (26), the upper end of which projects above the top of the plug (25), in order that it may be sealed by the resilient sealing member (30).

**Patentansprüche**

1. Bürettenvorrichtung zum Eingeben einer Flüssigkeit in den Körper eines Patienten, bestehend aus einem Bürettenrohr (21) in das Flüssigkeit an einem oberen Ende einfüllbar ist und aus dem Flüssigkeit an einem unteren Ende in die Eingangsöffnung einer dünnen hohlen Kanüle (26) auszuströmen vermag, wobei das Rohr (21) mit einem napfförmigen Ansatz (22) versehen ist, der einen Teil des Bodens (23) des Rohres bildet und durch den Flüssigkeit aus dem Rohr (21) in die Kanüle (26) zu strömen vermag, einem Schwimmerventilteil (28) innerhalb des Bürettenrohrs (21) und aus einer Einrichtung (35) zur Begrenzung der nach oben gerichteten Schwimmerbewegung des Ventilteils (28) derart, daß es in seiner oberen Stellung noch in dem unteren Abschnitt des Rohres (21) ist, wobei die Anordnung so getroffen ist, daß Ventilteil (28) in seiner unteren Stellung die Flüssigkeitsströmung in die Kanüle (26) durch Abdichten des Eingangs zu der Kanüle (26) innerhalb des napfförmigen Ansatzes (22) unterbricht, wenn der Flüssigkeitspiegel in dem Rohr (21) auf einen vorbestimmten Pegel fällt, dadurch gekennzeichnet, daß das Schwimmerventilteil (28) einen im wesentlichen zylindrischen Ventilkörper aufweist, der passend innerhalb des Bürettenrohrs (21) sitzt, wobei er einen ringförmigen Raum (31) zwischen dem Außenrand des Teils (28) und der inneren Oberfläche des Rohrs (21) freiläßt, wodurch die Querschnittsfläche des Ventilkörpers im wesentlichen größer ist als die der hohlen Kanüle (26), und daß Ventilteil (28) einen nach unten weisenden Ventilschließabschnitt (29) mit einem Gummi oder einem anderen elastischen Dichtelement (30) besitzt, welches an der Unterseite im wesentlichen flach ist und durch welches die Eingangsöffnung der hohlen Kanüle (26) schließbar ist, wobei das elastische Abdichtelement (30) unmittelbar auf der Oberseite der Kanüle (26) derart ruht, daß sich die flache Unterseite des Abdichtelements (30) horizontal über die Eingangsöffnung der Kanüle (26) erstreckt.

2. Vorrichtung nach Anspruch 1, dadurch gekennzeichnet, daß der napfförmige Einsatz (22) eine zentrale Ausgangsöffnung (24) besitzt, welche mittels eines in einer Form oder dergleichen hergestellten Stopfens (25) verschlossen ist, in den die dünne hohle Kanüle (26) passend eingesetzt ist, wobei die Kanüle (26) oben und unten derart offen ist, daß Flüssigkeit innerhalb des Rohres (21) in die Kanüle (26) zu fließen vermag, deren oberes Ende über die Oberseite des Stopfens (25) für eine Abdichtung mittels des elastischen Abdichtelements (30) vorsteht.

**Revendications**

1. Burette pour administrer un liquide dans le corps d'un patient, comprenant un tube de burette (21) dans lequel un liquide peut être distribué dans une extrémité supérieure et dont le liquide peut s'écouler par une extrémité inférieure dans l'orifice d'entrée d'une mince canule (26), le tube (21) comportant une saillie (22) constituant une partie du fond (23) du tube et à travers laquelle un liquide peut s'écouler hors du tube (21) dans la canule (26), un organe obturateur (28) de soupape, flottant, à l'intérieur du tube de burette (21) et des moyens (35) pour limiter le mouvement de montée de l'organe (28) dû à sa flottabilité, de sorte que, dans sa position haute, il se trouve toujours dans la partie inférieure du tube (21), l'agencement étant tel que lorsque le niveau du liquide dans le tube (21) tombe à un niveau prédéterminé, l'organe (28) dans sa position basse arrête l'écoulement du liquide dans la canule (26) en fermant l'entrée de la canule (26) à l'intérieur de la saillie (22) en forme de godet, caractérisée en ce que l'organe obturateur flottant (28) comprend un corps de soupape à peu près cylindrique qui s'ajuste à l'intérieur du tube de burette (21) en laissant un espace annulaire (31) entre le bord externe de l'organe (28) et la surface interne du tube (21) grâce à quoi la surface en section transversale du corps de soupape est notablement supérieure à celle de la canule creuse (26), et en ce que l'organe (28) comporte une partie (29) de fermeture s'étendant vers le bas comprenant un organe d'étanchéité (30) en caoutchouc, ou autre, élastique, qui est à peu près plat sur sa face inférieure et qui apte à fermer l'orifice d'entrée de la canule creuse (26) avec l'organe d'étanchéité élastique (30) reposant directement sur la partie supérieure de la canule (26) de manière que la surface inférieure plate de l'organe d'étanchéité (30) s'étende horizontalement en travers de l'orifice d'entrée de la canule (26).

2. Appareil suivant la revendication 1, caractérisé en ce que la saillie (22) en forme de godet comporte une ouverture centrale (24) de sortie, fermée au moyen d'un bouchon (25) moulé, ou autre, dans lequel est ajustée la mince canule creuse (26), cette dernière étant ouverte à ses parties supérieure et inférieure de façon qu'un liquide dans le tube (21) puisse s'écouler dans ladite canule (26), dont l'extrémité supérieure dépasse au-dessus de la partie supérieure du bouchon (25) afin qu'elle puisse être fermée par l'organe d'étanchéité élastique (30).

Fig. 1

Fig 3

Fig. 2

Fig. 4